# EUROPEAN PATENT SPECIFICATION

(11) **EP 3 991 757 B1**
(45) Date of publication and mention of the grant of the patent: **10.01.2024**
(21) Application number: 20205359.1
(22) Date of filing: 03.11.2020
(51) Int. Cl.: A61L 2/20, A61L 2/22, A61L 2/24, B67B 3/00, A61L 2/18

(54) **STERILIZATION APPARATUS FOR RECEPTACLE CLOSURES**
STERILISATIONSVORRICHTUNG FÜR BEHÄLTERVERSCHLÜSSEN
APPAREIL DE STÉRILISATION DE FERMETURES DE RÉCEPTACLE

(43) Date of publication of application: 04.05.2022
(73) Proprietor: Sidel Participations, 76930 Octeville-sur-Mer (FR)
(72) Inventor: BORGESE, Rossana, 43126 Parma (IT); CENCI, Mattia, 43126 Parma (IT); PAMBIANCHI, Matteo, 43126 Parma (IT)
(74) Representative: Sidel Group

(56) References cited:
- EP-A1- 2 546 155
- EP-A1- 3 391 911
- EP-A1- 3 670 404
- JP-A- 2015 527 270

## Description

### TECHNICAL FIELD

The present invention relates to a sterilization apparatus for receptacle closures, in particular receptacle closures for receptacles being filled with a pourable product.

Advantageously, the present invention further relates to method of sterilizing receptacle closures, in particular receptacle closures for receptacles being filled with a pourable product.

### BACKGROUND ART

The sterilization of packaging material, in particular within the food packaging sector, is of fundamental interest for guaranteeing the needed shelf life of the packaged products and, accordingly, the safety of the consumers. This is even more important when food products are packaged under aseptic conditions.

It is known in the art to fill any type of pourable product, in particular any type of pourable food product such as carbonated liquids (e.g. sparkling water, soft drinks and beer), non-carbonated liquids (including still water, juices, teas, sport drinks, wine, etc) and beverages containing pulps into receptacles, such as containers, vessels, jars and bottles made of base components, like glass, plastics, aluminum, steel, and composites.

In general, the receptacles prior to being filled with the pourable product are sterilized within a receptacle sterilization apparatus and are subsequently filled with the desired pourable product within a filling apparatus.

After the filling of the receptacles, typically the respective pouring openings of the receptacles are closed by the application and fastening of respective receptacle closures.

Prior to the application of the receptacle closures, the receptacle closures must be sterilized within a respective sterilization apparatus. After sterilization the sterile receptacle closures are fed to a capping apparatus, which also receives the filled receptacles.

A drawback of known sterilization apparatuses resides in the lacking possibility of adopting its processing velocities in dependence of the processing conditions of the capping apparatus.

A further drawback of such known sterilization apparatuses is that due the increased temperatures of the receptacle closures, the receptacle closures are more susceptible to deformations due to the forces acting within the succession of receptacle closures.

Therefore, with particular reference to EP-A-3391911 and EP-A-3670404, each disclosing a sterilization apparatus comprising a guide sector with a guide rail for sterilizing multiple receptacle closures in succession, the Applicant has developed an alternative sterilization apparatus, which is based on a conveying device, which comprises a guide track arranged within an isolation chamber and an endless track, which carries a plurality of moveable cart assemblies. Each cart assembly is operatively coupled to a respective pushing element, which is designed to interact with a respective group of a limited number of receptacle closures placed on the guide track so as to advance the respective group along the advancement path defined by the guide track.

In one embodiment, the endless track is arranged below the isolation chamber and each moveable cart is provided with a first interaction member. The conveying device further comprises a plurality of auxiliary carts arranged within the isolation chamber and moveably coupled onto a rail. Each auxiliary cart is associated to one respective cart and comprises a respective second interaction element configured to interact with the respective first interaction element so as to transfer advancement of the respective cart to an advancement of the auxiliary cart.

Even though such sterilization apparatuses work satisfyingly well, a desire is felt to further improve the known sterilization apparatuses and/or the methods of sterilization.

### DISCLOSURE OF INVENTION

It is therefore an object of the present invention to provide in a straightforward and low-cost manner an improved sterilization apparatus for receptacle closures.

It is another object of the present invention to provide in a straightforward and low-cost manner an improved method of sterilizing receptacle closures.

According to the present invention, there are provided sterilization apparatuses and a method for sterilizing receptacle closures according to the independent claims.

Further advantageous embodiments are specified in the respective dependent claims.

### BRIEF DESCRIPTION OF THE DRAWINGS

A non-limiting embodiment of the present invention will be described by way of example with reference to the accompanying drawings, in which:
Figure 1 is a schematic top view of a sterilization apparatus according to the present invention, with parts removed for clarity;
Figure 2 is a section view of the sterilization apparatus of Figure 1, with parts removed for clarity;
Figure 3 is an enlarged section view of details of the sterilization apparatus of Figure 1, with parts removed for clarity;
Figure 4 is an enlarged perspective view of some details of the sterilization apparatus of Figure 1, with parts removed for clarity;
Figure 5 is an enlarged top view of some details of the sterilization apparatus of Figure 1, with parts removed for clarity; and
Figure 6 is an enlarged perspective view of some more details of the sterilization apparatus of Figure 1, with parts removed for clarity.

### BEST MODES FOR CARRYING OUT THE INVENTION

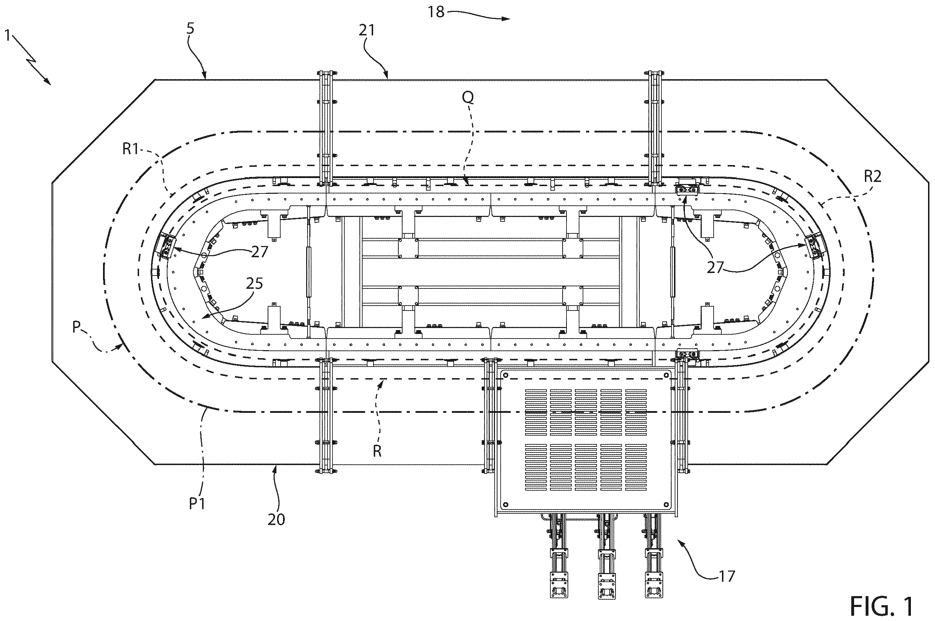

With particular reference to Figure 1, reference number 1 indicates as a whole a sterilization apparatus for sterilizing receptacle closures 2.

In particular, receptacle closures 2 are designed to be applied onto receptacles, such as bottles, jars, vessels, containers or the like, in particular being made of base components, like glass, paper or cardboard, plastics, aluminum, steel, and composites.

Preferentially, the receptacles are designed to contain a pourable product, in particular a pourable food product, such as carbonated liquids (e.g. sparkling water, soft drinks and beer), non-carbonated liquids (including still water, juices, teas, sport drinks, wine, milk, etc.), emulsions and beverages containing pulps.

Moreover, receptacle closures 2 may be of the type known as crown corks, screw caps, sports caps, stoppers or similar, and they may be produced from a variety of materials such as plastics and metal. It is further known that the receptacle closures 2 may vary in format (i.e. in size and/or dimensions).

In the specific example disclosed, the type of receptacle closures 2 are threaded receptacle closures, in particular plastic screw caps, to be screwed onto the receptacles. However, it must be clear that the present invention may be also used to particular advantage for any other type of receptacle closures 2 such as crown corks, sports caps, stoppers and others made also from materials different than plastic, such as metal or cork.

According to some possible embodiments, sterilization apparatus 1 could be part of a packaging machine configured to package pourable products such as pourable food products into the receptacles. In particular, according to such an embodiment sterilization apparatus 1 is configured to sterilize receptacle closures 2 and to feed the sterilized receptacle closures 2 to a capping apparatus of the packaging machine, the capping apparatus being configured to apply the sterilized receptacle closures 2 onto the receptacles.

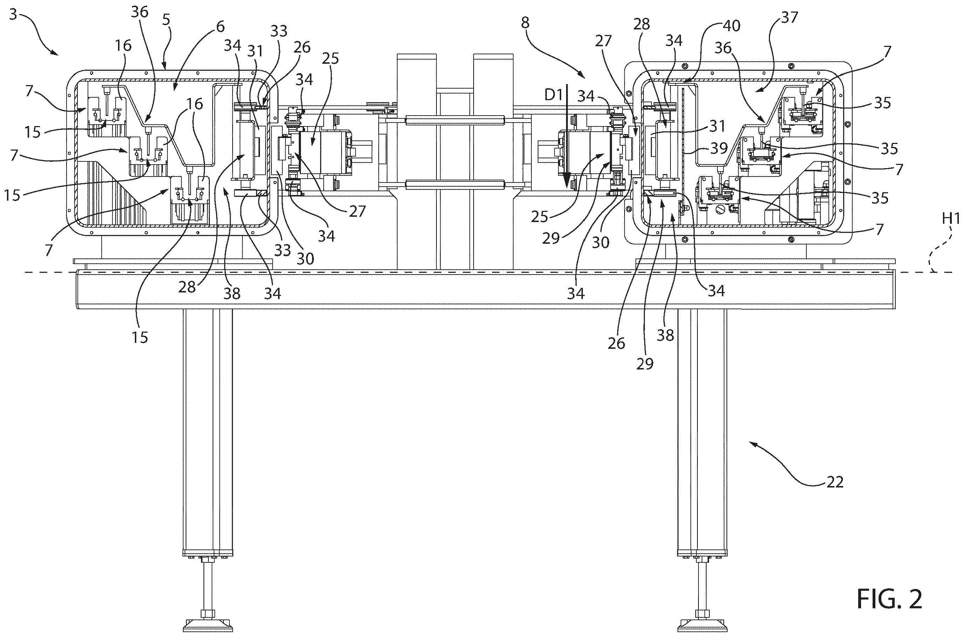

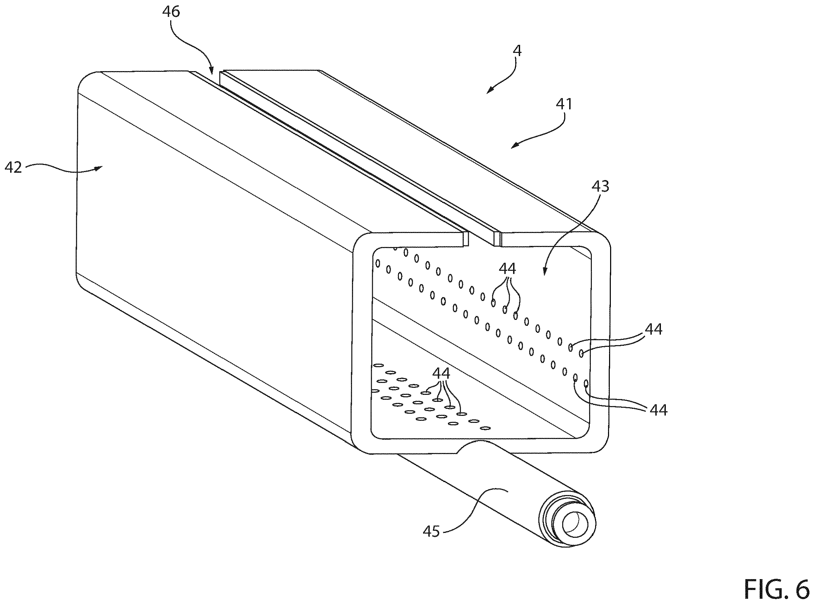

With particular reference to Figures 1 to 6, sterilization apparatus 1 comprises:
- a conveying device 3 (see in particular Figures 2 to 5) configured to advance receptacle closures 2 along an advancement path P;
- a sterilization unit 4 (only partially shown in Figure 6) configured to sterilize receptacle closures 2 during advancement of the receptacle closures 2 along at least a sterilization portion P1 of advancement path P; and
- an isolation chamber 5, in particular an endless isolation channel, having an inner space 6, in particular a sterile and/or aseptic inner space 6, housing at least a portion of conveying device 3 so that receptacle closures 2 advance, in use, within inner space 6 when advancing, in use, along advancement path P.

With particular reference to Figures 2 to 4, conveying device 3 comprises:
- at least one or more guide 7, in the specific example shown three, arranged within inner space 6 and configured to support and/or guide receptacle closures 2 and defining and/or delimiting advancement path P; and
- an advancement unit 8 configured to advance receptacle closures 2 along advancement path P and/or guide track 7.

In more detail, each guide track 7 comprises an inlet 9 for receiving receptacle closures 2 and an outlet (not shown) for releasing receptacle closures 2 from the respective guide track 9. In particular, advancement path P extends between inlet 9 and the outlet; i.e. conveying device 3 is configured to advance receptacle closures 2 between inlet 9 and the outlet.

Preferentially, each guide track 7 is configured to support and/or guide specific types and/or formats of receptacle closures 2, in particular different from the ones of the other guide tracks 7. In such way, sterilization apparatus 1 is adapted to handle a significant number of different types and/or formats of receptacles closures 2 without the need of any format changes when changing the format and/or type of receptacle closures 2 to be treated.

Additionally or in addition, guide tracks 7 could be used simultaneously for increasing the productivity of sterilization apparatus 2.

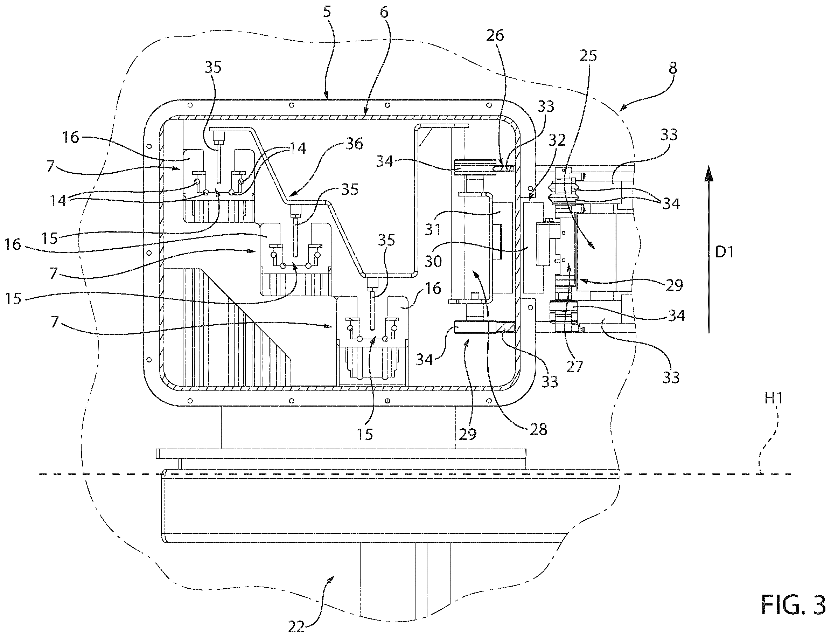

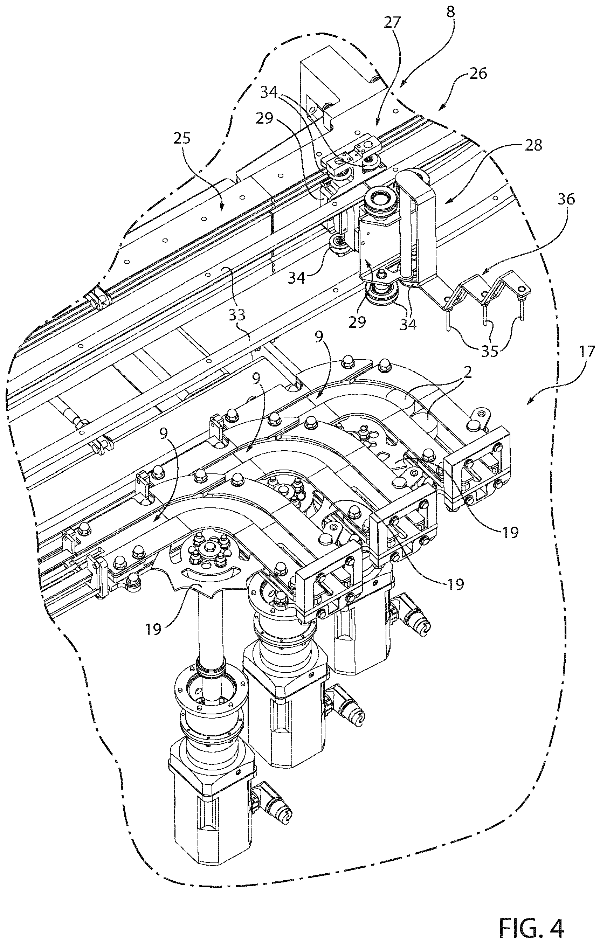

In further detail and with particular reference to Figures 3 and 4, each guide track 7 comprises a plurality of guide bars 14 for supporting and/or guiding receptacle closures 2 and delimiting and/or defining an advancement space 15 within which receptacle closures 2 advance, in use, along advancement path P.

Preferentially, each guide track 7 also comprises a support assembly 16 (having a plurality of support elements) for supporting guide bars 14.

According to some preferred non-limiting embodiments, each guide track 7 comprises a plurality of linear and curved portions so as to define linear and curved portions of advancement path P.

Preferentially, guide tracks 7 are parallel to one another and in particular displaced, in particular laterally displaced, from one another. Furthermore, guide tracks 7 are arranged on differing elevations.

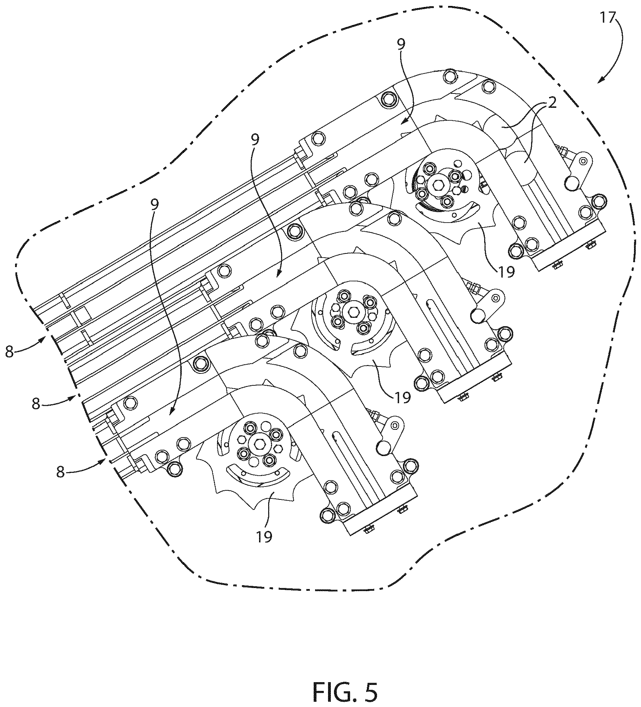

With particular reference to Figures 4 and 5, conveying device 3 also comprises a feed unit 17 configured to feed receptacle closures 2 into guide tracks 7.

In more detail, feed unit 17 comprises one or more feeding channels 18, in particular each one connected to one respective guide track 7, and one or more feeders, in particular one or more feeding star wheels 19, each one configured to advance receptacle closures 2 within the respective feeding channel 18 and towards and/or into the respective guide track 7.

Preferentially, feeding star wheel 19 is adapted to at least partially control the feeding speed of receptacle closures 2.

With particular reference to Figures 1 to 3, isolation chamber 5 separates inner space 6 from an (hostile) outer environment 18.

Moreover, sterilization apparatus 1 also comprises a conditioning device (not shown) operationally connected to isolation chamber 5 and configured to control an ambient condition within inner space 6. In particular and as is described in further detail below, the conditioning device is configured to at least locally control the pressure and/or the temperature and/or the humidity and/or the sterility and/or the cleanliness within inner space 6.

In more detail, isolation chamber 5 comprises an inlet opening and an outlet opening allowing for the feeding of receptacle closures 2 respectively into and out from inner space 6. In particular, feed unit 17 is arranged at the inlet opening.

Furthermore, the outlets of guide tracks 7 are arranged in the area of and/or at the outlet opening.

Additionally, isolation chamber 5 comprises at least a sterilization zone 20 and a clean zone 21 arranged downstream from and in particular directly connected to sterilization zone 20 along advancement path P. In particular, sterilization zone 20 and contact zone 21 delimit respective portions of inner space 6.

Advantageously, sterilization zone 20 comprises at least an injection portion and a contact portion arranged downstream from the injection portion along advancement path P.

Preferentially, sterilization unit 4 is configured to inject a sterilizing agent, such as hydrogen peroxide and/or any other chemical sterilizing agent in gaseous, vaporous and/or liquid form, into sterilization zone 20, in particular the injection portion. In more detail, in use, a cloud of sterilizing agent develops within at least the injection portion, in particular so that the sterilizing agent may deposit on receptacle closures 2 during advancement along sterilization portion P1 and within the injection portion.

Furthermore, in use, during advancement of receptacle closures 2 within the contact portion the sterilizing agent is in contact with (having been deposited onto) and acts on receptacle closures 2. In particular, the concentration of the sterilizing agent within the contact portion is (significantly) lower than the one within the injection portion.

Furthermore, the conditioning device is configured to maintain a determined temperature within at least the contact portion so as to guarantee activation of the sterilizing agent.

In particular, in use, receptacle closures 2 advance along sterilization portion P1, when advancing within sterilization zone 20.

Preferentially, sterilization zone 20 also comprises a pre-heating portion arranged upstream from the injection portion along advancement path P. Furthermore, the conditioning unit may be configured to control the temperature within pre-heating portion so as to pre-heat receptacle closures 2 during advancement within the pre-heating portion.

In further detail, clean zone 21 comprises a drying and venting portion so that during advancement of receptacle closures 2 within the drying and venting portion the sterilizing agent present on receptacle closures 2 dries of and/or evaporates from receptacle closures 2. For this reason, sterilization apparatus 1 preferentially comprises a venting unit configured to vent and/or extract gas from the drying and venting portion. In particular, the venting unit is configured to remove (substantially) any residues of the sterilizing agent being present within the drying and venting portion.

In particular, by providing for the drying and venting portion it is possible to guarantee that receptacle closures 2 are (substantially) void of any residues of the sterilizing agent when exiting sterilization apparatus 1, in particular isolation chamber 5.

According to a preferred non-limiting embodiment, clean zone 21 also comprises a cooling portion being provided with means for an active and/or passive cooling of receptacle closures 2 during, in use, advancement of receptacle closures 2 within the cooling portion. In particular, the cooling portion is arranged downstream from the drying and venting portion along advancement path P.

In further detail, isolation chamber 5 is designed such that inner space 6 extends at least between the inlet opening and the outlet opening and preferentially also between outlet opening and inlet opening (i.e. inner space 6 has an endless configuration). According to the embodiment disclosed isolation housing 5 and consequently inner space 6 presents an endless configuration.

Furthermore, isolation chamber 5 presents linear and curved portions.

With particular reference to Figure 1, sterilization apparatus 1 also comprises a support structure 22 carrying at least conveying device 3 and isolation chamber 5.

In more detail, support structure 22 is mountable onto a floor surface of a production plant within which sterilization apparatus 1 is placed and defines a support plane H1 from which isolation chamber 5 and/or conveying device 3 erect. In particular, support plane H1 has a (substantially) horizontal orientation and/or is parallel to the floor surface.

Advantageously, advancement unit 8 is based on linear motor technology.

With particular reference to Figures 2, 3 and 4, advancement unit 8 comprises at least:
- a first (endless) track 25;
- a second (endless) track 26 laterally spaced apart from, and in particular parallel to, track 25;
- a plurality of first (independent) cart assemblies 27 moveably coupled to and onto track 25 and configured to advance along track 25;
- an actuation assembly configured to control advancement of cart assemblies 27 along track 25, in particular independently from one another; and
- a plurality of second cart assemblies 28 moveably coupled onto track 26 and each one being operatively coupled to one respective cart assembly 27, in particular by means of a magnetic and/or electromagnetic interaction, such that advancement of the respective cart assembly 27 along track 25 actuates, in use, advancement of the corresponding cart assembly 28 along track 25.

In particular, laterally spaced apart from indicates a displacement between track 25 and track 26 along a direction having a (substantially) horizontal orientation.

Moreover, the term independent with respect to cart assemblies 27 indicates that, in use, advancement of each cart assembly 27 occurs independently from the other cart assemblies 27. Even more particular, each cart assembly 27 may be accelerated and decelerated independently from the other cart assemblies 27. Furthermore, as each cart assembly 28 is operatively coupled to one respective cart assembly 27, also each cart assembly 28 advances, in use, independent from the other cart assemblies 28 and may be accelerated and decelerated independently from the other cart assemblies 28.

According to some possible embodiments, track 25 and cart assemblies 27 define an actuation device configured to control advancement of cart assemblies 28 along track 26.

Advantageously, as track 25 and track 26 are laterally spaced apart from one another also each cart assembly 27 and the respective cart assembly 28 are laterally spaced apart from (and adjacent to) one another.

Moreover, as track 25 and track 26 are laterally spaced apart from one another, track 25 and track 26 define an interspace. In addition, each cart assembly 27 is arranged within the interspace (and accordingly is arranged such to advance, in use, within the interspace).

Moreover, a wall portion of isolation chamber 5 may be arranged within the interspace.

Advantageously, track 25 is laterally spaced apart from isolation chamber 5 (i.e. track 25 is arranged outside of inner space 6; in other words, track 25 is arranged within outer environment 18) and track 26 is arranged within inner space 6 (i.e. within isolation chamber 5).

Furthermore, one of isolation chamber 5 and track 25 surrounds the other one of track 25 and isolation chamber 5. In the specific embodiment disclosed, isolation chamber 5 surrounds track 25.

In more detail, track 25 defines a respective (endless) advancement path Q of cart assemblies 28 and track 26 defines a respective (endless) advancement path R of cart assemblies 28 parallel to advancement path Q. In particular, one of advancement path R and advancement path Q surrounds the other one of advancement path Q and advancement path R, in the specific case shown advancement path R surrounds advancement path Q.

According to the specific embodiment shown, track 25 and track 26 present linear and curved portions.

With particular reference to Figures 2, 3 and 4, each cart assembly 27 and each cart assembly 28 comprises a respective cart 29 moveably coupled to respectively track 25 and track 26. Moreover, each cart 29 of cart assemblies 27 is adjacent to and/or laterally spaced apart from one respective cart 29 of cart assemblies 28, in particular leaving a space between one another within which is placed a wall portion of isolation chamber 5.

Moreover, each cart 29 of cart assemblies 27 comprises a respective first interaction element 30 and each cart 29 of cart assemblies 28 comprises a respective second interaction element 31. In particular, each interaction element 30 and the respective interaction element 31 are configured to magnetically and/or electromagnetically interact with one another for operatively coupling the respective cart assembly 27 and the respective cart assembly 28 with one another.

In more detail, each interaction element 30 and each interaction element 31 comprise, in particular consist of, at least one magnet and/or one ferromagnetic component and/or one electromagnetic coil.

According to the specific embodiment shown, each interaction element 30 and the respective interaction element 31 interpose a wall portion of isolation chamber 5 between one another.

In particular, isolation chamber 5 may comprise a respective (annular) groove 32 and each interaction element 31 is arranged within groove 32 and/or is designed to advance, in use, within groove 32.

In more detail, each one of track 25 and track 26 comprises a pair of rails 33 spaced apart along a direction D1, in particular having a (substantially) vertical orientation, each pair of rails 33 being designed to guide advancement of the respective carts 29 along the corresponding track 25 or 26.

Preferentially, each cart 29 comprises a plurality of wheels 34, each coupled to and configured to run along one respective rail 33.

Furthermore, the respective carts 29 of cart assemblies 27 also comprise an interaction member, e.g. of the magnetic or ferromagnetic type, configured to interact with the actuation assembly allowing for the control of advancement of carts assemblies 27 along track 25.

According to some preferred non-limiting embodiments, the actuation assembly is configured to control the advancement, in particular the respective advancement speeds, of each cart assembly 27 and thereby of the respective cart assemblies 28, in particular of the respective carts independently from the other ones. In particular, in use, the advancement speed of cart assemblies 27 and consequently of cart assembly 28 is not necessarily constant and can be subject to accelerations and decelerations.

Moreover, the actuation assembly may comprise a plurality of coils for selectively forming an electromagnetic field and being positioned (adjacent and) along track 25. In particular, the interaction members are configured to interact with the electromagnetic field.

Advantageously and with particular reference to Figures 2 to 4, each cart assembly 28 comprises at least one pushing element 35, preferentially a plurality of pushing elements 35, configured to interact with at least one receptacle closure 2, preferentially with a group of receptacle closures 2, for advancing receptacle closures 2 or the group of receptacle closures 2 along advancement path P, in particular during advancement along at least a portion of track 26. In particular, each pushing element 35 is coupled to the respective cart 29.

In particular, each pushing element 35 advances, in use, together with its respective cart assembly 28 so as to push the respective receptacle closures 2 along advancement path P.

In particular, each pushing element 35 is associated to one respective guide track 7, in particular so as to push the respective receptacle closure 2 or the respective group of receptacle closure 2 along the respective guide track 7.

In particular, in use, cart assemblies 28 advance along an operative portion R1 of advancement path R when the respective pushing elements 35 engage with the respective receptacle closure 2 or the respective group of receptacle closures 2. In particular, operative portion R1 is parallel to advancement path P. Even more particular, each pushing element 35 is configured to start to engage and disengage from the respective receptacle closure 2 or the respective group of receptacle closures 2 respectively at inlet 9 and the outlet.

Moreover, cart assemblies 28 advance, in use, along a return portion R2 (downstream of operative portion R1 along advancement path R) so as to return cart assemblies 28 to operative portion R1.

According to some preferred non-limiting embodiments, each pushing element 35 is configured to engage with a group of receptacle closures 2 successively arranged on guide track 7 and/or within advancement space 15, in particular so as to advance the respective group along advancement path P upon advancement of the respective cart assembly 28 along track 26 and as actuated by advancement of the respective cart assembly 27 along track 25.

Preferentially but not necessarily, each pushing element 35 is configured to engage with and/or contact at least the receptacle closure 2 of the respective group, which is arranged upstream from the others along advancement path P, so as to apply a pushing force on the respective group. According to some preferred non-limiting embodiments, each group comprises not more than 30, in particular between 5 to 20, even more particular between 5 to 15, receptacle closures 2. In particular, this allows to reduce the forces acting on receptacle closures 2, thereby e.g. limiting the risk of deforming receptacle closures 2.

It should be noted that according to some possible embodiments, the number of receptacle closures 2 within a group of receptacle closures 2 may be adapted according to the required number of sterilized receptacle closures 2 (per hour) and/or as needed by the capping apparatus.

In addition, each cart assembly 28 also comprises a coupling structure 36, in particular having a first section carrying and/or supporting the respective pushing element 35 and a second section opposed to the first section and being connected to the respective cart 29, in particular for mechanically coupling the respective pushing element 35 to the respective cart 29. According to the non-limiting embodiment shown, the respective first section of each coupling structure 36 carries the plurality of pushing elements 35.

According to some preferred non-limiting embodiments, the actuation assembly is configured to selectively control the advancement speed of each cart assembly 27 and therefore of the respective cart assembly 28 in dependence of the local position of each cart assembly 27 on track 25 and therefore in dependence of the local position of the respective cart assembly 28 on track 26.

Additionally, actuation assembly may also be configured to control advancement of cart assemblies 27 and 28 in dependence of the specific type and/or format of receptacle closure 2, in particular to optimize the sterilization of receptacle closures 2. In particular, actuation assembly may control and vary the residence-time receptacle closures 2 should remain within the varying portions of isolation chamber 5, in particular within sterilization zone 20 and clean zone 21. It should be noted that the residence-time is defined as the through-put time of receptacle closures 2 within sterilization zone 20 and clean zone 21.

Moreover, the actuation assembly is configured to control advance of cart assemblies 28 such that the residence-time of receptacle closures 2 within the pre-heat portion and/or the injection portion and/or the contact portion and/or the drying and venting portion corresponds to desired and/or needed times.

In particular, actuation assembly is also configured to control advancement of cart assemblies 28 so as to guarantee that sterilization apparatus 1 is adapted to sterilize a required number of receptacle closures 2 (which may depend on the operation and the requirements of the capping apparatus).

Advantageously and with particular reference to Figures 2 and 3, isolation chamber 5, in particular sterilization zone 20 and even more particular also clean zone 21, comprises at least a chamber portion 37, a chamber portion 38 and a partition wall 39 separating chamber portion 37 from chamber portion 38. While guide track 7 is arranged within chamber portion 37, carts 29 of cart assemblies 28 are positioned such to advance within chamber portion 38 and/or at least a portion of track 26 is arranged within chamber portion 38.

In particular, chamber portions 37 and 38 are parallel to one another.

It should be noted that chamber portions 37 and 38 may have an annular configuration or an arc-shaped configuration.

Moreover, each cart assembly 28 may be arranged such that the respective first section is positioned so as to advance within chamber portion 37 and the respective second section is mounted such so as to advance within chamber portion 38.

Additionally, partition wall 39 defines, in particular together with other wall portions of isolation chamber 5, and/or comprises a slit 40.

Furthermore, each coupling structure 36 comprises an intermediate section connecting the respective first section and the respective second section with one another. In particular, each intermediate section is arranged such to extend through slit 40. In other words, each intermediate section is designed to partially advance within chamber portion 37 and to partially advance within chamber portion 38.

According to some preferred non-limiting embodiments, the conditioning device is configured to control a first pressure within chamber portion 37 and a second pressure within chamber portion 38 distinct from the first pressure. Advantageously, the first pressure is larger than the second pressure.

In more detail, the conditioning device comprises a suction device fluidically (and directly) connected to second chamber portion 38. In particular, the suction device is configured to create a flow of gas from chamber portion 37 to chamber portion 38 and out of chamber portion 38.

With particular reference to Figure 6, sterilization unit 4 comprises an injection assembly 41 configured to inject the sterilizing agent, in particular in gaseous, vaporous and/or liquid form, into at least a section of inner space 6, in particular at the injection portion.

In further detail, injection assembly 41 comprises a delimiting housing 42 arranged around a portion of the guide track 7, in particular the portion of guide track 7 being positioned within sterilization zone 20, even more particular within the injection portion, and delimiting a sterilization space 43. In particular, delimiting housing 42 allows to reduce the volume of sterilization space 43 with respect to the one of inner space 6 and/or the respective portion attributable to chamber portion 37.

Furthermore, injection assembly 41 is designed to inject the sterilizing agent into sterilization space 43. In use, a cloud of the sterilizing agent develops within sterilization space 43.

In particular, therefore, delimiting housing 42 comprises a plurality of injection openings 44 through which, in use, the sterilizing agent enters sterilization space 43.

In further detail, delimiting housing 42 may comprise a hollow space being in fluidic connection with injection openings 44.

In even further detail, delimiting housing 42 has a rectangular or quadratic cross-section. Alternatively, delimiting housing 42 could have a circular or oval cross-section.

Moreover, delimiting housing 42 may comprises a plurality of walls, in particular being perpendicularly arranged to one another. In the example shown, delimiting housing 42 comprises three main walls and two partially walls.

Furthermore, injection assembly 42 also comprises a delivery tube 45 designed to deliver and/or direct the sterilizing agent into the hollow space.

In even further detail, injection assembly 41 also comprises a sterilizing agent conditioning unit being fluidically connected to delivery tube 45 and being configured to condition the sterilizing agent for injection.

Moreover, delivery tube 45 may be integral to delimiting housing 42 or delimiting housing 42 may comprise delivery tube 45.

According to some possible embodiments not shown, delivery tube 45 may be configured to also directly inject at least some parts of the sterilizing agent into sterilization space 43.

According to the specific example shown, delivery tube 45 is arranged below guide track 7.

Preferentially, delimiting housing 42 comprises a slit 46, in particular defined between the two partial walls, designed to allow for pushing elements 35 to enter into sterilization space 43.

In use, sterilization apparatus 1 sterilizes receptacle closures 2, in particular prior to being applied onto filled receptacles.

More precisely, the sterilization of receptacle closures 2 comprises at least the steps of:
- advancing receptacle closures 2 along advancement path P; and
- sterilizing receptacle closures 2 during advancement along sterilization portion P1.

Furthermore, the sterilization of receptacle closures 2 may also comprise the step of maintaining the receptacle closures 2 under sterile and/or aseptic conditions after the step of sterilizing. In particular, during the step of maintaining, receptacle closures 2 advance within clean zone 21.

Furthermore, the sterilization of receptacle closures 2 may also comprise the step of feeding receptacle closures 2 onto one respective guide track 7, in particular as a function of the type and/or format of receptacle closures 2.

Moreover, during the step of feeding, at least one, preferentially more than one receptacle closure 2 is fed onto guide track 7. Preferentially, during the step of feeding, operation of feed unit 17 is such to place a group of successively arranged receptacle closures 2 on guide track 7 so that during the step of advancing the group of receptacle closures 2 advance as a group along advancement path P.

Advantageously, the step of advancing also comprises the sub-steps of:
- advancing cart assemblies 28 along track 26 and/or advancement path R; and
- pushing of one receptacle closure 2 or a group of successively arranged receptacles closures 2 by means of one respective pushing element 35 along advancement path P as a result of the advancement of the respective cart assembly 28.

In more detail, during the sub-step of advancing cart assemblies 28, the (selective) advancement of the respective cart assemblies 27 along advancement path Q and/or track 25 is (selectively) controlled, in particular by means of the operation of actuation assembly, even more particular by selectively controlling the coils arranged along track 25.

Moreover, during the sub-step of advancing cart assemblies 28, cart assemblies 28 may be accelerated and decelerated, in particular by respectively accelerating and decelerating the respective cart assemblies 27, in order to guarantee desired and/or required residence-times of receptacle closures 2 within the varying portions and/or zones (e.g. sterilization zone 20 and/or clean zone 21 and/or the injection portion and/or the contact portion and/or the pre-heating portion and/or the drying and venting portion and/or the cooling portion) of isolation chamber 5. In other words, during the sub-step of advancing cart assemblies 28, the advancement speed of each cart assembly 28, in particular the respective cart 29, is locally controlled.

In particular, such control occurs in dependence of the type and/or format of receptacle closures 2.

In particular, it should be noted, that sterilization apparatus 1 may be connected to a capping apparatus, which operates at a determined operation velocity. Therefore, sterilization apparatus 1 must deliver the required number of sterilized receptacle closures 2 to the capping apparatus. Thus, in order to optimize the sterilization process, the residence-time in the varying portions and/or zones of isolation chamber 5 is controlled such to obtain the desired sterilization effect and to guarantee the needed flow of sterilized receptacle closures 2 to the capping apparatus.

According to some possible embodiments, in dependence of the required number of sterilized receptacle closures 2 to be delivered to the capping apparatus, it may be possible to adjust the number of receptacle closures 2 within a group of successively arranged receptacle closures 2.

Alternatively or in addition, the number of cart assemblies 27 and the number of cart assemblies 28 being arranged on respectively track 25 and track 26 is a function of the required number of sterilized receptacle closures 2 (per hour) and/or the type and/or format of receptacle closures 2.

In further detail, during the step of sterilizing, the sterilizing agent is injected into inner space 6, in particular into the respective portion of inner space 6 associated to the injection portion.

Moreover, during the step of sterilizing, the sterilizing agent is introduced into sterilization space 43. In more detail, the sterilizing agent exits from injection openings 44 into sterilization space 43 during advancement of receptacle closures 2 through sterilization space 43. In even more detail, the sterilizing agent is delivered by delivery tube 45 and enters the hollow space of delimiting housing 42 from which it enters sterilization space 45.

According to some preferred non-limiting embodiments, during the sterilization of receptacle closures 2, also a step of conditioning is executed, during which the condition within inner space 6, in particular within chamber portion 37 and chamber portion 38 is controlled.

In more detail, during the step of conditioning, at least the first pressure and the second pressure are controlled, in particular so that the first pressure is larger than the second pressure.

In even more detail, during the step of conditioning, the suction device generates a flow of gas from chamber portion 37 into chamber portion 38 and out of chamber portion 38.

According to another aspect, the sterilization apparatus 1 comprises a guide sector 7.

For each closure 2, the closure 2 is sterilized during a movement of the closure 2 along the guide sector 7.

The apparatus 1 comprises a sterile and/or aseptic chamber 5. The guide sector 7 is located within the chamber 5. The apparatus 1 comprises a first track sector 25 located outside with respect to the chamber 5. The apparatus 1 comprises a plurality of first carts 27. The first carts 27 are mechanically independent from each other. The apparatus 1 comprises a second track sector 26 located within the chamber 5. The apparatus comprises a plurality of second carts 28. The second carts 28 are mechanically independent from each other. The apparatus 1 comprises a plurality of pushers 35. For each first cart 27, the first cart 27 can be subjected to a movement which occurs along the first track sector 25 and outside the chamber 5. For each second cart 28, the second cart 28 can be subjected to a movement which occurs along the second track sector 26 and within the chamber 5. For each pusher 35, the pusher 35 can be subjected to a movement along the guide sector 7 and within the chamber 5. For each pusher 35, the movement of the pusher 35 corresponds to or causes the movement of at least one closure 2, by means of the pusher 35 pushing the at least one closure 2. For each second cart 28, the movement of the second cart 28 corresponds to or causes the movement of a respective pusher 35, by means of a mechanical coupling between the second cart 28 and the pusher 35. For each first cart 27, the movement of the first cart 27 corresponds to or causes the movement of a respective second cart 28, by means of magnetic and/or electromagnetic interaction between the first cart 27 and the second cart 28. The guide sector 7 and the first track sector 25 are located on mutually opposite sides of the second track sector 26. In this way, it is lowered and/or avoided the risk of a possible blockage of the closures 2 along the guide 7 causing the derailment of the first cart 27 with respect to the first track portion 25. Therefore, it is improved the mechanical robustness of a sterilization apparatus for sterilizing closures 2 which are for closing container adapted to contain a pourable product, wherein the apparatus is more simple from a mechanical point of view and less subjected to possible wear of the components, thanks to the electromagnetic interaction between the first carts 27 and the second carts 28, and wherein it is lowered or avoided the risk of having mechanical problems due to a possible blockage of the closures 2 while they are advanced along the guide sector 7.

From another point of view, the first track sector 25, the second track sector 26 and the guide sector 7 are located one after the other along a direction oriented transversally to the movements of the respective closures or oriented transversally to the guide sector 7.

From another point of view, the apparatus 1 is configured so that, for each second cart 28, the second cart 28 is located spatially between the second track sector 26 and the guide sector 7.

From another point of view, the second track sector 26 is spatially interposed between the first track sector 25 and the guide sector 7.

With this spatial configuration, a bending moment acting on a second cart 28 due to a blockage of the closures 2 along the guide sector 7, which bending moment is transferred to the respective first cart 27 through the magnetic and/or electromagnetic interaction between the second cart 28 and the first cart 27, will not tend to cause the derailment of the first cart 27 with respect to the first track sector 25.

The apparatus comprises a partition wall 39 located within the chamber 5 to divide the chamber 5 in at least a first chamber portion 37 and a second chamber portion 38. The partition wall 39 defines a slot 40 which is interposed between an edge of the partition wall 39 and a surface portion of the wall of the chamber 5. The first chamber portion 37 and the second chamber portion 38 are in fluid communication with each other through the slot 40. The guide sector 7 is located within the first chamber portion 37. The second track sector 26 is located within said second chamber portion 38. For each second cart 29, the movement of the second cart 28 occurs within the second chamber portion 38. For each pusher 35, the movement of the pusher 35 occurs within the first chamber portion 37. Each second cart 28 is mechanically coupled to the respective pusher 35 by means of a respective coupling structure 36. For each second cart 28, the coupling structure 36 extends between the first chamber portion 37 and the second chamber portion 38 by passing through the slot 40. The apparatus comprises a conditioning device for controlling control an ambient condition within the chamber 5. The conditioning device is configured so that a first pressure P1 within the first chamber portion 37 is kept greater than and a second pressure P2 within the second chamber portion 38, by means of pneumatic interaction between said conditioning device and said chamber 5. In this way it is improved also the ability of the apparatus in maintaining a sterile and/or aseptic condition of the guide 7 where the closures 2 are advanced.

For each closure 2, the closure 2 is sterilized by introducing a sterilization agent in the guide sector 7. The apparatus 1 comprises a jacket 42 located within the chamber 5 and enclosing the guide sector 7. The jacket 42 defines a coating or a cladding for the guide sector 7. The guide sector 7 is located in a sterilization space 43 defined by the jacket 42. The jacket 42 defines a plurality of openings 44. The openings 44 are faced into said sterilization space 43 so as to be in fluid communication with said guide sector 7. The sterilization agent is introduced in the guide sector 7 by means of inputting the sterilization agent in the jacket 42 and by means of the jacket 42 outputting the sterilization agent through said openings 44. In this way it is improved the repeatability of the sterilizing apparatus as regards the sterilization phase of the closures 2, thanks to the jacket 42 being configured for better concentrating the sterilization agent to lower or avoid the dispersing of the sterilization agent.

The apparatus 1 comprises a delivery tube 45 which is in fluid communication with the jacket 42 for inputting said sterilization agent in said jacket 42. The jacket 42 has a longitudinal development along the longitudinal development of the guide sector 7. The delivery tube 45 extends longitudinally along the longitudinal development of the jacket 42. In this way it is improved the uniformity of distribution of the sterilization agent along the guide sector 7.

The jacket 42 is located within the first chamber portion 37. The jacket 42 defines a slit 46 located on the periphery of the sterilization space 43. The pusher 35 is inserted in the guide sector 7 by passing through the slit 46. The slit 46 allows and/or guides the movement of the pusher 35 along the guide sector 7.

The advantages of sterilization apparatus 1 according to the present invention will be clear from the foregoing description.

In particular, as tracks 25 and 26 are laterally spaced from one another the stability of cart assemblies 27 are improved. In the configurations known in the art, in case of e.g. a blockage of receptacle closures 2 within guide track 7, cart assemblies 27 have been observed to derail. Such problems are avoided by the lateral spacing of tracks 25 and 26 from one another.

A further advantage resides in advancing cart assemblies 27 within outer environment 18 and cart assemblies 28 within inner space 6, improving thereby the sterilization process.

An even other advantage can be seen in arranging guide track(s) 7 within chamber portion 37 and advancing carts 29 of cart assemblies 38 within chamber portion 38. This allows to further increase the sterility of the portion of inner space 6 within which receptacle closures 2 advance.

An additional advantage resides in providing for delimiting housing 42, which allows to more precisely control the sterilization process as sterilization space 43 is smaller than inner space 6 and/or the portion of inner space 6 of chamber portion 37. This allows to more precisely control the concentration and/or the conditions of the sterilizing agent within sterilization space 43.

Clearly, changes may be made to sterilization apparatus 1 and the respective methods as described herein without, however, departing from the scope of protection as defined in the accompanying claims.

## Claims

1. A sterilization apparatus (1) for sterilizing receptacle closures (2), comprising:
- a guide sector (7), the apparatus (1) being configured, for each closure (2), so that the closure (2) is sterilized during a movement of the closure (2) along the guide sector (7), the apparatus being configured so that, for each closure (2), the closure is sterilized by introducing a sterilization agent in said guide sector (7);
- a sterile and/or aseptic chamber (5), the guide sector (7) being located within the chamber (5);
- a first track sector (25) located outside with respect to the chamber (5);
- a plurality of first carts (27) mechanically independent from each other;
- a second track sector (26) located within the chamber (5) ;
- a plurality of second carts (28) mechanically independent from each other;
- a plurality of pushers (35);
wherein the apparatus is configured so that:
- for each first cart (27), the first cart (27) can be subjected to a movement along the first track sector (25) and outside the chamber (5);
- for each second cart (28), the second cart (28) can be subjected to a movement along the second track sector (26) and within the chamber (5);
- for each pusher (35), the pusher (35) can be subjected to a movement along the guide sector (7) and within the chamber (5);
- for each pusher (35), the movement of the pusher (35) along the guide sector (7) corresponds to the movement of at least one closure (2) along the guide sector (7), by means of the pusher (35) pushing the at least one closure (2);
- for each second cart (28), the movement of the second cart (28) along the second track sector (26) corresponds to the movement of a respective pusher (35) along the guide sector (7), by means of a mechanical coupling between the second cart (28) and the pusher (35);
- for each first cart (27), the movement of the first cart (27) along the first track sector (25) corresponds to the movement of a respective second cart (28) along the second track sector (26), by means of magnetic and/or electromagnetic interaction between the first cart (27) and the second cart (28);
- the guide sector (7) and the first track sector (25) are located on opposite sides of the second track sector (26) .

2. Apparatus according to claim 1, wherein the first track sector (25), the second track sector (26) and the guide sector (7) are located one after the other along a direction oriented transversally to the movements of the respective closures or transversally to the guide sector (7).

3. Sterilization apparatus according to claim 1 or 2, wherein:
- the apparatus (1) comprises a partition wall (39) located within the chamber (5) to divide the chamber (5) in at least a first chamber portion (37) and a second chamber portion (38), the partition wall (39) defining a slot (40) which is interposed between an edge of the partition wall (39) and a surface portion of the wall of the chamber (5), the first chamber portion (37) and the second chamber portion (38) being in fluid communication with each other through the slot (40);
- the guide sector (7) is located within the first chamber portion (37);
- the second track sector (26) is located within said second chamber portion (38);
- the apparatus is configured so that, for each second cart (28), the movement of the second cart (28) along the second track sector (26) occurs within the second chamber portion (38);
- the apparatus is configured so that, for each pusher (35), the movement of the pusher (35) along the guide sector (7) occurs within the first chamber portion (37);
- each second cart (28) is mechanically coupled to the respective pusher (35) by means of a respective coupling structure (36), the coupling structure (36) extending between the first chamber portion (37) and the second chamber portion (38) by passing through the slot (40);
the apparatus comprises a conditioning device for controlling an ambient condition within the chamber (5);
wherein the conditioning device is configured so that a first pressure (P1) within the first chamber portion (37) is greater than and a second pressure (P2) within the second chamber portion (38), by means of pneumatically interaction between said conditioning device and said chamber (5).

4. Apparatus (1) according to any of the preceding claims, wherein:
- the apparatus (1) comprises a jacket (42) located within the chamber (5) and enclosing said guide sector (7), the guide sector (7) being located in a sterilization space (43) defined by the jacket (42);
- said jacket (42) defines a plurality of openings (44), said openings (44) being faced into said sterilization space (43) as to be in fluid communication with said guide sector (7) ;
- the apparatus is configured so that the sterilization agent is introduced in said guide sector (7) by means of inputting the sterilization agent in the jacket (42) and by means of the jacket (42) outputting the sterilization agent through said openings (44).

5. Apparatus (1) according to claim 4, comprising a delivery tube (45) in fluidic communication with said jacket (42) for inputting said sterilization agent in said jacket (42), wherein:
- the jacket (42) has a longitudinal development along the longitudinal development of the guide sector (7);
- the delivery tube (42) extends longitudinally along the longitudinal development of the jacket (42).

6. Apparatus (1) according to claim 4 or 5, wherein the jacket (42) defines a slit (46) located on the periphery of the sterilization space (43), the apparatus (1) being configured so that, for each pusher (35), the pusher (35) is inserted in the guide sector (7) by passing through the slit (46) and the slit (46) allows the movement of the pusher (35) along the guide sector (7).

## Patentansprüche

1. Sterilisationsvorrichtung (1) zur Sterilisierung von Behälterverschlüssen (2), umfassend:
- einen Führungssektor (7), wobei die Vorrichtung (1) für jeden Verschluss (2) ausgestaltet ist, so dass der Verschluss (2) während einer Bewegung des Verschlusses (2) entlang des Führungssektors (7) sterilisiert wird, wobei die Vorrichtung so ausgestaltet ist, dass der Verschluss für jeden Verschluss (2) durch Einführen eines Sterilisationsmittels in den Führungssektor (7) sterilisiert wird,
- eine sterile und/oder aseptische Kammer (5), wobei der Führungssektor (7) in der Kammer (5) angeordnet ist,
- einen ersten Bahnsektor (25), der bezüglich der Kammer (5) außerhalb angeordnet ist,
- eine Vielzahl von ersten Wagen (27), die mechanisch voneinander unabhängig sind,
- einen zweiten Bahnsektor (26), der in der Kammer (5) angeordnet ist,
- eine Vielzahl von zweiten Wagen (28), die mechanisch voneinander unabhängig sind,
- eine Vielzahl von Schiebern (35),
wobei die Vorrichtung so ausgestaltet ist, dass:
- der erste Wagen (27) für jeden ersten Wagen (27) einer Bewegung entlang des ersten Bahnsektors (25) und außerhalb der Kammer (5) unterworfen werden kann,
- der zweite Wagen (28) für jeden zweiten Wagen (28) einer Bewegung entlang des zweiten Bahnsektors (26) und innerhalb der Kammer (5) unterworfen werden kann,
- der Schieber (35) für jeden Schieber (35) einer Bewegung entlang des Führungssektors (7) und in der Kammer (5) unterworfen werden kann,
- die Bewegung des Schiebers (35) entlang des Führungssektors (7) für jeden Schieber (35) der Bewegung mindestens eines Verschlusses (2) entlang des Führungssektors (7) mittels des Schiebers (35), der den mindestens einen Verschluss (2) schiebt, entspricht,
- die Bewegung des zweiten Wagens (28) entlang des zweiten Bahnsektors (26) für jeden zweiten Wagen (28) der Bewegung eines jeweiligen Schiebers (35) entlang des Führungssektors (7) mittels einer mechanischen Koppelung zwischen dem zweiten Wagen (28) und dem Schieber (35) entspricht,
- die Bewegung des ersten Wagens (27) entlang des ersten Bahnsektors (25) für jeden ersten Wagen (27) der Bewegung eines jeweiligen zweiten Wagens (28) entlang des zweiten Bahnsektors (26) mittels eines magnetischen und/oder elektromagnetischen Zusammenwirkens zwischen dem ersten Wagen (27) und dem zweiten Wagen (28) entspricht,
- der Führungssektor (7) und der erste Bahnsektor (25) an gegenüberliegenden Seiten des zweiten Bahnsektors (26) angeordnet sind.

2. Vorrichtung nach Anspruch 1, wobei der erste Bahnsektor (25), der zweite Bahnsektor (26) und der Führungssektor (7) hintereinander entlang einer Richtung angeordnet sind, die quer zu den Bewegungen der jeweiligen Verschlüsse oder quer zu dem Führungssektor (7) ausgerichtet ist.

3. Sterilisationsvorrichtung nach Anspruch 1 oder 2, wobei:
- die Vorrichtung (1) eine in der Kammer (5) angeordnete Trennwand (39) zur Unterteilung der Kammer (5) in mindestens einen ersten Kammerabschnitt (37) und einen zweiten Kammerabschnitt (38) umfasst, wobei die Trennwand (39) einen Schlitz (40) definiert, der zwischen einem Rand der Trennwand (39) und einem Flächenabschnitt der Wand der Kammer (5) angeordnet ist, wobei der erste Kammerabschnitt (37) und der zweite Kammerabschnitt (38) durch den Schlitz (40) in Fluidverbindung miteinander sind,
- der Führungssektor (7) in dem ersten Kammerabschnitt (37) angeordnet ist,
- der zweite Bahnsektor (26) in dem zweiten Kammerabschnitt (38) angeordnet ist,
- die Vorrichtung so ausgestaltet ist, dass die Bewegung des zweiten Wagens (28) entlang des zweiten Bahnsektors (26) für jeden zweiten Wagen (28) in dem zweiten Kammerabschnitt (38) erfolgt,
- die Vorrichtung so ausgestaltet ist, dass die Bewegung des Schiebers (35) entlang des Führungssektors (7) für jeden Schieber (35) in dem ersten Kammerabschnitt (37) erfolgt,
- jeder zweite Wagen (28) mittels einer jeweiligen Kopplungsstruktur (36) mechanisch an den jeweiligen Schieber (35) gekoppelt ist, wobei sich die Kopplungsstruktur (36) zwischen dem ersten Kammerabschnitt (37) und dem zweiten Kammerabschnitt (38) erstreckt, indem sie durch den Schlitz (40) geht,
die Vorrichtung eine Konditionierungsvorrichtung zur Steuerung einer Umgebungsbedingung in der Kammer (5) umfasst,
wobei die Konditionierungsvorrichtung so ausgestaltet ist, dass ein erster Druck (P1) in dem ersten Kammerabschnitt (37) mittels pneumatischen Zusammenwirkens zwischen der Konditionierungsvorrichtung und der Kammer (5) höher als ein zweiter Druck (P2) in dem zweiten Kammerabschnitt (38) ist.

4. Vorrichtung (1) nach einem der vorhergehenden Ansprüche, wobei:
- die Vorrichtung (1) einen Mantel (42) umfasst, der in der Kammer (5) angeordnet ist und den Führungssektor (7) umschließt, wobei der Führungssektor (7) in einem von dem Mantel (42) definierten Sterilisationsraum (43) angeordnet ist,
- der Mantel (42) eine Vielzahl von Öffnungen (44) definiert, wobei die Öffnungen (44) in den Sterilisationsraum (43) weisen, um mit dem Führungssektor (7) in Fluidverbindung zu stehen,
- die Vorrichtung so ausgestaltet ist, dass das Sterilisationsmittel in den Führungssektor (7) eingeführt wird, indem das Sterilisationsmittel in den Mantel (42) eingeführt und mittels des Mantels (42) durch die Öffnungen (44) ausgetragen wird.

5. Vorrichtung (1) nach Anspruch 4, umfassend ein Zuführrohr (45) in Fluidverbindung mit dem Mantel (42) zum Einführen des Sterilisationsmittels in den Mantel (42), wobei:
- der Mantel (42) einen Längsverlauf entlang des Längsverlaufs des Führungssektors (7) hat,
- sich das Zuführrohr (42) in Längsrichtung entlang des Längsverlaufs des Mantels (42) erstreckt.

6. Vorrichtung (1) nach Anspruch 4 oder 5, wobei der Mantel (42) einen Schlitz (46) definiert, der an dem Umfang des Sterilisationsraums (43) angeordnet ist, wobei die Vorrichtung (1) so ausgestaltet ist, dass der Schieber (35) für jeden Schieber (35) in den Führungssektor (7) eingeführt wird, indem er durch den Schlitz (46) geht, und der Schlitz (46) die Bewegung des Schiebers (35) entlang des Führungssektors (7) gestattet.

## Revendications

1. Appareil de stérilisation (1) permettant de stériliser des fermetures de contenant (2), comprenant :
- un secteur de guidage (7), l'appareil (1) étant configuré, pour chaque fermeture (2), de sorte que la fermeture (2) soit stérilisée lors d'un déplacement de la fermeture (2) le long du secteur de guidage (7), l'appareil étant configuré de sorte que, pour chaque fermeture (2), la fermeture soit stérilisée par introduction d'un agent de stérilisation dans ledit secteur de guidage (7) ;
- une chambre stérile et/ou aseptique (5), le secteur de guidage (7) étant situé à l'intérieur de la chambre (5) ;
- un premier secteur de piste (25) situé à l'extérieur par rapport à la chambre (5) ;
- une pluralité de premiers chariots (27) mécaniquement indépendants les uns des autres ;
- un second secteur de piste (26) situé à l'intérieur de la chambre (5) ;
- une pluralité de seconds chariots (28) mécaniquement indépendants les uns des autres ;
- une pluralité de poussoirs (35) ;
dans lequel l'appareil est configuré de sorte que :
- pour chaque premier chariot (27), le premier chariot (27) peut être soumis à un mouvement le long du premier secteur de piste (25) et à l'extérieur de la chambre (5) ;
- pour chaque second chariot (28), le second chariot (28) peut être soumis à un mouvement le long du second secteur de piste (26) et à l'intérieur de la chambre (5) ;
- pour chaque poussoir (35), le poussoir (35) peut être soumis à un mouvement le long du secteur de guidage (7) et à l'intérieur de la chambre (5) ;
- pour chaque poussoir (35), le mouvement du poussoir (35) le long du secteur de guidage (7) correspond au mouvement d'au moins une fermeture (2) le long du secteur de guidage (7), au moyen du poussoir (35) poussant l'au moins une fermeture (2) ;
- pour chaque second chariot (28), le mouvement du second chariot (28) le long du second secteur de piste (26) correspond au mouvement d'un poussoir (35) respectif le long du secteur de guidage (7), au moyen d'un accouplement mécanique entre le second chariot (28) et le poussoir (35) ;
- pour chaque premier chariot (27), le mouvement du premier chariot (27) le long du premier secteur de piste (25) correspond au mouvement d'un second chariot (28) respectif le long du second secteur de piste (26), au moyen d'une interaction magnétique et/ou électromagnétique entre le premier chariot (27) et le second chariot (28) ;
- le secteur de guidage (7) et le premier secteur de piste (25) sont situés sur des côtés opposés du second secteur de piste (26).

2. Appareil selon la revendication 1, dans lequel le premier secteur de piste (25), le second secteur de piste (26) et le secteur de guidage (7) sont situés l'un après l'autre le long d'une direction orientée transversalement aux mouvements des fermetures respectives ou transversalement au secteur de guidage (7).

3. Appareil de stérilisation selon la revendication 1 ou 2, dans lequel :
- l'appareil (1) comprend une paroi de séparation (39) située à l'intérieur de la chambre (5) pour diviser la chambre (5) en au moins une première partie de chambre (37) et une seconde partie de chambre (38), la paroi de séparation (39) définissant une fente (40) qui est interposée entre un bord de la paroi de séparation (39) et une partie de surface de la paroi de la chambre (5), la première partie de chambre (37) et la seconde partie de chambre (38) étant en communication fluidique l'une avec l'autre à travers la fente (40) ;
- le secteur de guidage (7) est situé à l'intérieur de la première partie de chambre (37) ;
- le second secteur de guidage (26) est situé à l'intérieur de ladite seconde partie de chambre (38) ;
- l'appareil est configuré de sorte que, pour chaque second chariot (28), le mouvement du second chariot (28) le long du second secteur de piste (26) se produise à l'intérieur de la seconde partie de chambre (38) ;
- l'appareil est configuré de sorte que, pour chaque poussoir (35), le mouvement du poussoir (35) le long du secteur de guidage (7) se produise à l'intérieur de la première partie de chambre (37) ;
- chaque second chariot (28) est mécaniquement accouplé au poussoir (35) respectif au moyen d'une structure d'accouplement (36) respective, la structure d'accouplement (36) s'étendant entre la première partie de chambre (37) et la seconde partie de chambre (38) en passant à travers la fente (40) ;
l'appareil comprend un dispositif de conditionnement permettant de réguler une condition ambiante à l'intérieur de la chambre (5) ;
dans lequel le dispositif de conditionnement est configuré de sorte qu'une première pression (P1) à l'intérieur de la première partie de chambre (37) soit supérieure à une seconde pression (P2) à l'intérieur de la seconde partie de chambre (38), au moyen d'une interaction pneumatique entre ledit dispositif de conditionnement et ladite chambre (5).

4. Appareil (1) selon l'une quelconque des revendications précédentes, dans lequel :
- l'appareil (1) comprend une chemise (42) située à l'intérieur de la chambre (5) et enfermant ledit secteur de guidage (7), le secteur de guidage (7) étant situé dans un espace de stérilisation (43) défini par la chemise (42) ;
- ladite chemise (42) définit une pluralité d'ouvertures (44), lesdites ouvertures (44) étant orientées face audit espace de stérilisation (43) de manière à être en communication fluidique avec ledit secteur de guidage (7) ;
- l'appareil est configuré de sorte que l'agent de stérilisation soit introduit dans ledit secteur de guidage (7) au moyen de l'entrée de l'agent de stérilisation dans la chemise (42) et au moyen de la chemise (42) sortant l'agent de stérilisation à travers lesdites ouvertures (44).

5. Appareil (1) selon la revendication 4, comprenant un tube de distribution (45) en communication fluidique avec ladite chemise (42) pour introduire ledit agent de stérilisation dans ladite chemise (42), dans lequel :
- la chemise (42) présente un développement longitudinal le long du développement longitudinal du secteur de guidage (7) ;
- le tube de distribution (42) s'étend longitudinalement le long du développement longitudinal de la chemise (42).

6. Appareil (1) selon la revendication 4 ou 5, dans lequel la chemise (42) définit une fente (46) située sur la périphérie de l'espace de stérilisation (43), l'appareil (1) étant configuré de sorte que, pour chaque poussoir (35), le poussoir (35) soit inséré dans le secteur de guidage (7) en traversant la fente (46) et la fente (46) permette le mouvement du poussoir (35) le long du secteur de guidage (7).
